(19) **Europäisches Patentamt**
**European Patent Office**
**Office européen des brevets**

(11) **EP 3 024 407 B1**

(12) **EUROPÄISCHE PATENTSCHRIFT**

(45) Veröffentlichungstag und Bekanntmachung des Hinweises auf die Patenterteilung:
**09.09.2020 Patentblatt 2020/37**

(51) Int Cl.:
***A61B 18/20*** (2006.01)

(21) Anmeldenummer: **13744965.8**

(22) Anmeldetag: **23.07.2013**

(86) Internationale Anmeldenummer:
**PCT/EP2013/002190**

(87) Internationale Veröffentlichungsnummer:
**WO 2015/010705 (29.01.2015 Gazette 2015/04)**

(54) **VORRICHTUNG ZUR THERMOKOAGULATION MITTELS LASERSTRAHLUNG**

DEVICE FOR THERMOCOAGULATION BY MEANS OF LASER RADIATION

APPAREIL DE COAGULATION THERMIQUE PAR RAYONNEMENT LASER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.06.2016 Patentblatt 2016/22**

(73) Patentinhaber: **Fraunhofer-Gesellschaft zur Förderung der angewandten Forschung e.V.
80686 München (DE)**

(72) Erfinder:
• **WEHNER, Martin
52134 Herzogenrath (DE)**
• **ADEN, Mirko
52068 Aachen (DE)**

(74) Vertreter: **Gagel, Roland
Patentanwaltskanzlei
Dr. Roland Gagel
Landsberger Strasse 480 a
81241 München (DE)**

(56) Entgegenhaltungen:
**WO-A2-2011/143663     US-A1- 2007 179 484
US-A1- 2012 296 238     US-A1- 2012 296 323**

• **ILAN GABAY ET AL: "Temperature-controlled two-wavelength laser soldering of tissues", LASERS IN SURGERY AND MEDICINE, Bd. 43, Nr. 9, 17. Oktober 2011 (2011-10-17), Seiten 907-913, XP055076662, ISSN: 0196-8092, DOI: 10.1002/lsm.21123**

**Beschreibung**

**Technisches Anwendungsgebiet**

Die vorliegende Erfindung betrifft eine Vorrichtung zur Thermokoagulation mittels Laserstrahlung, die mindestens zwei Laserstrahlquellen mit unterschiedlicher Zentralwellenlänge emittieren, eine Einrichtung zur Überlagerung der Laserstrahlung der Laserstrahlquellen und eine Steuereinrichtung zur Steuerung der Laserleistung der Laserstrahlquellen aufweist.

Eine derartige Vorrichtung lässt sich beispielsweise für das sog. Lasergewebeschweißen (LTW: laser tissue welding), das Verbinden von Gewebe unter Verwendung eines Proteinklebers (LTS: laser tissue soldering), die Laserfixierung von Wundauflagen, das Kleben von Gefäßen, das Versiegeln von Wundflächen oder die Blutstillung und Koagulation von Weichgewebe einsetzen.

Der Ersatz von Nähten und Klammern durch Lasergewebeschweißen oder -kleben mit oder ohne Zusatzmaterialien ermöglicht sofort flüssigkeitsdichte Verbindungen von Hohlorganen. Der laserunterstützte Wundverschluss ermöglicht eine keimdichte Versiegelung von Organ- und Körperflächen und liefert ein hervorragendes kosmetisches Ergebnis ohne Narbenbildung durch Stich- und Klammermarken.

**Stand der Technik**

Das Lasergewebeschweißen (LTW) und das Verbinden von Gewebe unter Verwendung von Proteinkleber (LTS) wurden bereits intensiv für unterschiedliche Anwendungen wie den Verschluss von Hautschnitten, die Fixierung von Wundauflagen, das Verschließen von Blutgefäßen und das Aufkleben von Pflaster auf Harnleiter, Blase und Hirnhaut untersucht. In den letzten Jahren wurde LTW in der Augenchirurgie als Ersatz für das Nähen erprobt. LTW beruht auf der Absorption von Laserstrahlung und der Erwärmung der Gewebe, die oberhalb von Temperaturen von 60°C zu einer thermischen Koagulation von Proteinen und einer Vernetzung von Kollagenfasern führt. Werden zusätzlich Proteinlösungen aufgebracht, wird das Verfahren als LTS bezeichnet. Üblicherweise werden die Proteinlösungen aus humanem Serumalbumin (HSA) oder für Tierexperimente aus bovinem Serumalbumin (BSA), mit einem Massenanteil von 30 bis 45% Albumin gelöst in Wasser, hergestellt.

Für die Thermokoagulation von Gewebe mittels Laserstrahlung werden bevorzugt Laserstrahlquellen mit Wellenlängen im nahen Infrarotbereich von etwa 780 bis 1064 nm eingesetzt. In diesem Wellenlängenbereich ist die Eindringtiefe im Gewebe relativ hoch, so dass Gewebsschichten bis zu einigen mm Dicke durchdrungen werden können. Da die Absorption von Proteinlösungen im sichtbaren und infraroten Spektralbereich der Absorption von Wasser und damit von Gewebe entspricht, ist eine selektive Erwärmung des Klebers ohne gleichzeitige Erwärmung des umliegenden Gewebes kaum möglich. Daher wurde vorgeschlagen, dem Kleber zusätzliche Absorber beizufügen, um die Absorption der Strahlung im Kleber zu erhöhen. Die Konzentration der absorbierenden Chromophore wird dabei so gewählt, dass die optische Eindringtiefe etwa der Dicke der Kleberschicht entspricht und so eine homogene Koagulation und gute Haftung auf dem Gewebe möglich wird.

Als zusätzlicher Absorber wurde hierbei der Farbstoff Indocyaningrün (ICG) zum Verschluss von Hautwunden im Tiermodell untersucht. Allerdings bestehen starke Vorbehalte in Bezug auf den Einsatz von ICG als extrinsischer Absorber in Proteinklebern, da zelltoxische Wirkungen auftreten könnten, die die Wundheilung verzögern und Entzündungsreaktionen hervorrufen können.

Aus diesem Grunde gibt es Überlegungen, durch Wahl anderer Laserwellenlängen eine höhere intrinsische Absorption des Gewebes zu erzielen und so die optische Eindringtiefe an die Schichtdicke des Gewebes anzupassen. Für eine möglichst gleichmäßige Erwärmung des Gewebes und zur Reduzierung von Temperaturgradienten wird in der US 2007/0179484 A1 eine Technik beschrieben, bei der gleichzeitig mehrere Laserstrahlquellen mit unterschiedlichen Wellenlängen zur Erwärmung des Gewebes bei der Thermokoagulation eingesetzt werden. Über einen Infrarot-Detektor wird die Temperatur der Gewebeoberfläche während des Prozesses gemessen und die Laserstrahlleistung der eingesetzten Laser zur Einstellung und Aufrechterhaltung einer gewünschten Temperatur geregelt. In einem Ausführungsbeispiel wird demonstriert, dass beim gleichzeitigen Betrieb von zwei Lasern unterschiedlicher Zentralwellenlänge eine höhere stabile Temperatur erzielt werden kann als bei Betrieb nur eines Lasers. Eine derartige Technik ist auch in I. Gabay et al., "Temperature-Controlled Two-Wavelength Laser Soldering of Tissues", Lasers in Surgery and Medicine 43:907-913 (2011) beschrieben. Zur Temperaturregelung wird die Laserleistung der beiden Laser dabei in analoger Weise variiert, so dass das Verhältnis der Laserleistungen jeweils gleich bleibt.

Aus US 2012296323, US 2003109860 und WO 2011/143663 A2 sind medizinische Laserbehandlungsvorrichtungen bekannt, die mit zwei Lasern unterschiedlicher Wellenlänge arbeiten und die die Gewebetemperatur mit Detektoren wie z.b Infrarot-Detektoren erfassen.

## Darstellung der Erfindung

Die Aufgabe der vorliegenden Erfindung besteht darin, eine Vorrichtung zur Thermokoagulation mittels Laserstrahlung anzugeben, mit der eine über die Tiefe möglichst gleichmäßige Thermokoagulation erreicht werden kann.

Die Aufgabe wird mit der Vorrichtung gemäß Patentanspruch 1 gelöst. Vorteilhafte Ausgestaltungen der Vorrichtung sind Gegenstand der abhängigen Patentansprüche oder lassen sich der nachfolgenden Beschreibung sowie dem Ausführungsbeispiel entnehmen.

Die vorgeschlagene Vorrichtung zur Thermokoagulation mittels Laserstrahlung weist mindestens zwei Laserstrahlquellen auf, die Laserstrahlung mit unterschiedlicher Zentralwellenlänge emittieren, eine Einrichtung zur Überlagerung der Laserstrahlung der Laserstrahlquellen sowie eine Steuereinrichtung zur Steuerung der Laserleistung der Laserstrahlquellen, die die Laserstrahlquellen in einem Betriebsmodus, hier als Bearbeitungsmodus bezeichnet, zur gleichzeitigen Emission der Laserstrahlung für die Durchführung eines Thermokoagulationsprozesses ansteuert. Die Vorrichtung zeichnet sich dadurch aus, dass die Steuereinrichtung das Verhältnis der Laserleistungen der gleichzeitig betriebenen Laserstrahlquellen im Bearbeitungsmodus nach einem vorgegebenen Verlauf und/oder in der Abhängigkeit von einem aus dem Koagulationsprozess erfassten Prozesssignal mit der Zeit ändert. Der vorgegebene Verlauf ist dabei vorzugsweise in einem Speicher hinterlegt, auf den die Steuereinrichtung Zugriff hat.

Unter der Thermokoagulation mittels Laserstrahlung sind hierbei vor allem Laserverfahren zu verstehen, bei denen Gewebe thermisch denaturiert wird, Gewebeteile durch Denaturierung und Vernetzung von Kollagenfasern verbunden werden, zusätzlich eingebrachte Proteine als Haftvermittler wirken oder Wundauflagen durch einen der Prozesse fixiert werden. Insbesondere sind damit die in der Beschreibungseinleitung genannten Techniken umfasst.

Bei der vorgeschlagenen Vorrichtung wird während der Durchführung des Thermokoagulationsprozesses das Verhältnis der Laserleistungen der eingesetzten Laserstrahlquellen unterschiedlicher Wellenlänge verändert. Hierbei wurde berücksichtigt, dass sich durch die dynamisch veränderlichen Gewebseigenschaften bei der Thermokoagulation die effektive optische Eindringtiefe verringert. So ändern sich insbesondere Absorption und Streuung aufgrund der thermischen Koagulation und der Austrocknung des Gewebes. Die Absorption nimmt leicht ab, die Streuung jedoch stark zu. Die Stärke dieser Veränderungen hängt von der Gewebeart und dessen Hämoglobin- und Wassergehalt ab und unterscheidet sich für die unterschiedlichen Wellenlängen der Laserstrahlung. Die Änderung des Verhältnisses der Laserleistungen der eingesetzten Laserstrahlquellen unterschiedlicher Wellenlänge während des Bearbeitungs- bzw. Thermokoagulationsprozesses bei gleichzeitiger Laseremission beider Laserstrahlquellen ermöglicht eine dynamische Anpassung an die dynamisch veränderlichen Streu- und Absorptionseigenschaften des Gewebes, die die optische Eindringtiefe während der Bearbeitung verändern. Damit lässt sich eine über den Thermokoagulationsprozess annähernd gleiche effektive optische Eindringtiefe erreichen.

Der Strahlungstransport im Schichtsystem Wundauflage - Kleber - Gewebe kann durch den Absorptionskoeffizienten $\mu_a$, den Streukoeffizienten $\mu_s$ und den Anisotropiefaktor g beschrieben werden, der die Vorzugsrichtung der Streuung angibt. Die Abschwächung eines Laserstrahls einer Wellenlänge $\lambda$ mit der Intensität I entlang seiner Ausbreitungsrichtung x kann mit dem Extinktionskoeffizienten $\mu_{ex}$ beschrieben werden:

$$I(x) = I_0 \cdot \exp(-\mu_{ex} \cdot x) \; ;$$

$$\mu_{ex} := \mu_s + \mu_a \; .$$

Wird ein streuendes und absorbierendes Materialsystem mit gerichteter Laserstrahlung beaufschlagt, so wird ein Teil der Strahlung gestreut und ein Teil absorbiert. Die gestreute Strahlung hat eine andere Richtung als die eingestrahlte. Die an einer Stelle im Material umgesetzte optische Energie Q ist proportional zum Absorptionskoeffizienten

$$Q = \mu_a \left( I_{0,Laser} \exp(-\mu_{ex} \cdot x) + F_s \right) \, ,$$

wobei $I_{0,Laser}$ den Energiefluss der Laserstrahlung an der Oberfläche der Probe und $F_s$ den Energiefluss der Streustrahlung darstellen.

Im Limes $\mu_s \to 0$ verschwindet auch der Term $F_s$ und der Term Q nimmt exponentiell mit der Koordinate x ab. Ist $\mu_s$ größer als $\mu_a$, nimmt die gerichtete Laserstrahlung in der Probe über eine kürzere Distanz ($1/\mu_{ex}$) ab, die Streustrahlung kann aber tiefer ins Material eindringen.

Wichtig bei der Koagulation von Proteinklebern ist die vollständige Durchhärtung der Kleberschicht, damit die maximale Festigkeit des Klebers und eine gute Adhäsion auf dem Gewebe erzielt werden können. Sinnvollerweise erfolgt die Auswahl der Laserwellenlänge in Abhängigkeit der Absorptionseigenschaften des Proteinklebers so, dass die effektive Absorptionslänge an die Dicke des Schichtsystems angepasst ist.

Da der Kleber zuerst in flüssigem Zustand eine schwach absorbierende Flüssigkeit mit vernachlässigbarer Streuung darstellt, wird die optische Eindringtiefe im Wesentlichen durch den effektiven Absorptionskoeffizienten beschrieben:

$$\mu_{eff}(1) \approx \sqrt{3} \cdot \mu_a$$

Wenn Proteinkleber wie BSA-Lösungen koagulieren, entsteht aus der klaren Flüssigkeit ein stark streuendes Material in gelartigem oder festem Zustand. Dieser Vorgang ist analog dem Braten eines Spiegeleis, wobei sich das klare Eiweiß zu einem kompakten, opaken Material verändert. Durch die starke Zunahme der Streuung in der Kleberschicht nimmt die effektive Schwächung zu und die effektive optische Eindringtiefe $1/\mu_{eff}$ ab:

$$\mu_{eff}(2) = \left[3\mu_a(\mu_a + \mu_s(1-g))\right]^{1/2} \text{ mit } g > 0 \text{ und } \mu_s \geq \mu_a ;$$

Dadurch ändert sich die Energieeinkopplung in den Kleber, so dass nur noch eine dünne oberflächennahe Schicht erwärmt wird. Die ursprüngliche Absorptionslänge $\mu_{eff}(1)$ kann aber wieder erzielt werden, wenn der Absorptionskoeffizient $\mu_a$ soweit reduziert wird, dass $\mu_{eff}(2)$ dem Anfangswert $\mu_{eff}(1)$ entspricht. Dazu kann die Wellenlänge der Laserstrahlung geändert werden, so dass eine geringere Absorption im teilweise koagulierten Kleber erzielt wird und die effektive optische Eindringtiefe zunimmt. In gleicher Weise kann dieser Effekt durch eine Änderung der spektralen Verteilung der eingesetzten Laserstrahlung erreicht werden, indem das Verhältnis der Laserleistungen der eingesetzten Laserstrahlquellen unterschiedlicher Wellenlänge verändert wird.

Durch die Änderung des Verhältnisses der Laserleistungen bei der vorgeschlagenen Vorrichtung während des Bearbeitungsprozesses lässt sich die spektrale Verteilung der Laserstrahlung an den sich verändernden Koagulationszustand anpassen. Dabei werden die Laserstrahlquellen vorzugsweise so gewählt, dass die Zentralwellenlänge einer der Laserstrahlquellen von dem biologischen Gewebe deutlich stärker absorbiert wird als die Zentralwellenlänge einer anderen eingesetzten Laserstrahlquelle. Eine der Wellenlängen ist damit gut zur Erwärmung der nicht koagulierten Schicht, die andere zur gleichmäßigen Erwärmung der koagulierten Schicht geeignet. Die Änderung des Verhältnisses der Laserleistungen ermöglicht eine variable Mischung der beiden Wellenlängen bei der Bearbeitung. Dadurch kann die effektive optische Eindringtiefe in einem weiten Bereich eingestellt werden, da die einzelnen spektralen Komponenten unterschiedliche Absorptions- und Streukoeffizienten im Gewebe und Kleber aufweisen. Mit Fortschreiten des Koagulationsprozesses ändern sich die Streueigenschaften der Kleberschicht, die Streuung nimmt zu. Dadurch wird die effektive Eindringtiefe der Strahlung verringert und die Laserstrahlung wird nun in kleineren Schichtdicken absorbiert. Dies kann zu dem oben beschriebenen Nachteil einer nicht vollständigen Koagulation und einer ungenügenden Haftung auf dem Gewebe führen. Durch Änderung des Verhältnisses der Laserleistungen der beiden Laserstrahlquellen mit den unterschiedlichen Zentralwellenlängen kann der Verringerung der effektiven optischen Eindringtiefe entgegengewirkt werden. Da die typischen Bestrahlungszeiten bei diesen Anwendungen im Bereich von etwa 0,2 s bis 10 s liegen, kann die spektrale Leistungsverteilung, die sich aus der Mischung der Laserstrahlung der beiden Laserstrahlquellen ergibt, innerhalb von Millisekunden genügend schnell an die geänderten Verhältnisse angepasst werden.

Die Anpassung bzw. die Änderung des Verhältnisses der Laserleistungen der Laserstrahlquellen im Bearbeitungsmodus, d. h. während der Bearbeitung des Gewebes, kann durch Vorgabe fester vorher bestimmter Werte bzw. eines festen zeitlichen Verlaufs der Laserleistungen erfolgen. Tritt bspw. die Trübung des Klebers nach typisch 1 s die Bestrahlungszeit auf, so wird nach 1 s der Anteil der stark absorbierenden Strahlung gegenüber der Laserstrahlung der anderen Laserstrahlquelle verringert. Je nach zu bearbeitendem Gewebe bzw. eingesetztem Kleber kann hierbei ein beliebiger zeitlicher Verlauf der Änderung des Verhältnisses der beiden Laserleistungen fest vorgegeben werden. Ein für die entsprechende Anwendung optimaler zeitlicher Verlauf des Verhältnisses kann durch Vorversuche ermittelt oder anhand einer Simulation abgeschätzt werden.

Die dynamische Anpassung des Verhältnisses der Laserleistungen kann auch anhand von Prozesssignalen erfolgen, die einen Hinweis auf geänderte Streueigenschaften während der Bearbeitung geben. Hierzu ist die Vorrichtung in einer vorteilhaften Ausgestaltung mit einer Einrichtung zur Erfassung von Rückstreusignalen von der bearbeiteten Oberfläche ausgestattet. Die Einrichtung, die einen geeigneten Detektor zur Erfassung der Stärke der rückgestreuten Laserstrahlung oder der Strahlung einer oder mehrerer zusätzlich eingesetzter Lichtquellen umfasst, liefert für die Steuereinrichtung ein Prozesssignal, anhand dessen die Steuereinrichtung das Verhältnis der Laserleistungen der beiden eingesetzten Laserstrahlquellen verändert. Die Abhängigkeit der Änderung des Verhältnisses von dem erfassten Rückstreusignal wird dabei vorgegeben. Eine geeignete Vorgabe für ein optimales Bearbeitungsergebnis kann wiederum durch Vorversuche oder durch eine Simulation vorab ermittelt werden. In einer einfachen Ausgestaltung wird das Verhältnis der Laserleistungen der beiden Laser so geändert, dass die Laserleistung der Laserstrahlquelle mit der stärker absorbierenden Zentralwellenlänge proportional zum erfassten Rückstreusignal verringert wird, während die gleichzeitig die Laserleistung der Laserstrahlquelle mit der geringer absorbierten Zentralwellenlänge proportional zum Rückstreusignal erhöht wird.

In einer Weiterbildung der Vorrichtung wird das Rückstreusignal spektral aufgelöst erfasst und analysiert. Auch die Erfassung und getrennte Auswertung des Rückstreusignals in mindestens zwei unterschiedlichen Spektralbereichen ist möglich. In beiden Fällen variiert die Steuereinrichtung auf Basis der spektralen Analyse der Rückstreuung bzw. der Stärke der in jedem Spektralbereich gemessenen Rückstreuung dann dynamisch das Verhältnis der Laserleistungen. Diese Berücksichtigung der spektralen Information bei der Rückstreuung ermöglicht eine genauere Anpassung der Laserleistungen an die veränderten Streueigenschaften. Zur Erweiterung des Wellenlängenbereiches der spektralen Auswertung können eine oder mehrere zusätzliche Lichtquellen, vorzugsweise mit größerer spektraler Bandbreite als die Laserstrahlquellen, eingesetzt werden, mit denen die Bearbeitungsoberfläche zur Erfassung und Analyse der Rückstreuung während der Bearbeitung beleuchtet wird.

In einer weiteren Ausgestaltung weist die Vorrichtung auch einen Temperaturdetektor auf, mit dem die Temperatur an der Oberfläche der Bearbeitungsstelle erfasst werden kann. Bei dem Temperatursensor kann es sich bspw. um einen IR-Detektor oder um ein Pyrometer handeln. In Abhängigkeit vom Temperatursignal regelt dann die Steuereinrichtung die Gesamtleistung der eingesetzten Laserstrahlquellen, um eine bestimmte Temperatur zu erreichen und/oder zu halten.

Als Laserstrahlquellen werden bei der vorgeschlagenen Vorrichtung vorzugsweise Diodenlaser bzw. Diodenlaserbarren eingesetzt, deren Laserstrahlung in eine Lichtleitfaser gekoppelt und so gleichzeitig und koaxial zur Bearbeitungs- bzw. Koagulationsstelle transportiert wird. Die Diodenlaser oder Diodenlaserbarren mit unterschiedlichen Zentralwellenlängen können dabei elektrisch unabhängig voneinander angesteuert werden, so dass eine schnelle Änderung der einzelnen Anteile bzw. des Verhältnisses der einzelnen Anteile innerhalb von Millisekunden möglich wird. Vorzugsweise erzeugt einer der Diodenlaser oder Diodenlaserbarren hierbei eine Wellenlänge von < 1250 nm, die von biologischem Gewebe nur schwach absorbiert wird, und ein anderer Diodenlaser bzw. Diodenlaserbarren eine Wellenlänge von > 1250 nm, die von biologischem Gewebe stark absorbiert wird.

Bevorzugte Wellenlängen bzw. Wellenlängenbereiche mit schwacher Absorption liegen zwischen 805 und 810 nm, bei 940 nm, zwischen 976 und 980 nm, bei 1064 nm sowie zwischen 1200 und 1230 nm. Für eine starke Absorption werden bevorzugt Wellenlängen zwischen 1320 und 1350 nm, zwischen 1460 und 1480 nm, zwischen 1520 und 1550 nm, bei 1700 nm oder bei 1940 nm eingesetzt. Für diese Wellenlängen bzw. Wellenlängenbereiche existieren bereits geeignete Diodenlaser bzw. Diodenlaserbarren oder -module.

Selbstverständlich lassen sich bei der vorgeschlagenen Vorrichtung nicht Laserstrahlquellen mit zwei unterschiedlichen Zentralwellenlängen sondern auch weitere Laserstrahlquellen einsetzen, die wiederum bei anderen Zentralwellenlängen emittieren. So kann bspw. ein Diodenlasermodul eingesetzt werden, das mit drei bei unterschiedlichen Wellenlängen emittierenden Emittern oder Barren bestückt ist, deren Strahlung dann gemeinsam in eine Lichtleitfaser eingekoppelt wird. Damit kann ggf. eine nochmals feinere dynamische Anpassung der effektiven optischen Einringtiefe an die sich ändernden Streu- und Absorptionseigenschaften des Gewebes erreicht werden.

**Kurze Beschreibung der Zeichnungen**

Die vorgeschlagene Vorrichtung wird nachfolgend anhand eines Ausführungsbeispiels in Verbindung mit den Zeichnungen nochmals näher erläutert. Hierbei zeigen:

Fig. 1    eine schematische Darstellung eines Beispiels der vorgeschlagenen Vorrichtung für die Thermokoagulation;

Fig. 2    ein Beispiel für die Variation der effektiven optischen Eindringtiefe durch Änderung des Leistungsverhältnisses zweier Laserstrahlquellen mit unter-schiedlichen Zentralwellenlängen für die Absorption von Laserstrahlung in Haut;

Fig. 3    ein Beispiel für das Remissionsspektrum bei breitbandiger Beleuchtung einer Gewebeprobe zu unterschiedlichen Zeiten einer Thermokoagulation;

Fig. 4    ein Beispiel für den Quotienten der Remission im Spektralbereich von 720 nm zu 550 nm mit zunehmender Koagulationsdauer; und

Fig. 5    ein Beispiel für die zeitliche Änderung der Remission im Wellenlängenintervall von 500 bis 550 nm mit zunehmender Koagulationsdauer.

**Wege zur Ausführung der Erfindung**

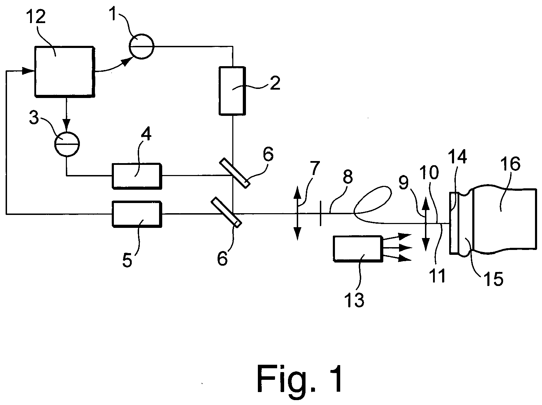

Figur 1 zeigt stark schematisiert ein Beispiel für eine Ausgestaltung der vorgeschlagenen Vorrichtung zur Thermokoagulation mittels Laserstrahlung. Die Vorrichtung umfasst in diesem Beispiel ein Diodenlasermodul mit zwei Diodenlaserbarren 2, 4 unterschiedlicher Wellenlänge, deren Strahlung über dichroitische Spiegel 6 und eine Koppeloptik 7 in eine Lichtleitfaser 8 eingekoppelt wird. Der Diodenlaserstrom kann über getrennt steuerbare Stromquellen 1, 3 für jeden Laserbarren 2, 4 getrennt eingestellt und damit die spektrale Leistungsverteilung am Bearbeitungsort gesteuert werden. Die Steuerung erfolgt über eine Steuereinrichtung 12, die in der Figur schematisch angedeutet ist.

Die Steuerung der Laserleistung der beiden Laserbarren 2, 4 und somit der spektralen Leistungsverteilung der in die Lichtleitfaser 8 eingekoppelten Laserstrahlung kann über die Bearbeitungsdauer fest vorgegeben sein, d. h. einem entsprechend programmierten Verlauf folgen, der in der Steuereinrichtung gespeichert wird. Die zeitliche Änderung der Leistungsanteile über die Bearbeitungs- bzw. Bestrahlungsdauer kann dabei bspw. anhand von Erfahrungswerten oder gezielter Vorexperimente oder Simulationen vorgegeben sein.

Im vorliegenden Beispiel erfolgt die Änderung des Verhältnisses der beiden Laserleistungen und somit die Änderung der spektralen Leistungsverteilung anhand eines Rückstreusignals 11 aus der Koagulationszone. Dazu ist die Vorrichtung mit einem Rückstreukanal und einem Detektor ausgestattet. Über den optischen Rückstreukanal wird das Rückstreusignal 11 koaxial zur Bearbeitungslaserstrahlung aus der Koagulationszone über einen dichroitischen Spiegel 6 zum Detektor 5 geführt. Die aus der Lichtleitfaser 8 austretende Bearbeitungsstrahlung 10 der Laserquellen wird dabei bspw. mit einer Kollimationsoptik 9 auf Körpergewebe 16 gerichtet, das mit einer Kleberschicht 15 und einer Wundauflage 14 bedeckt ist. Durch die Einwirkung der Laserstrahlung wird die Wundauflage mit dem Gewebe verbunden, um den Verschluss einer Wunde zu erreichen. Das erfasste Rückstreusignal 11 wird der Steuereinrichtung 12 zugeführt, die dann die Leistungsanteile der beiden Laserbarren 2, 4 in Abhängigkeit vom Rückstreusignal 11 verändert. Damit wird eine dynamische Anpassung der effektiven optischen Eindringtiefe an die veränderlichen Eigenschaften des Gewebes und der Wundauflage, insbesondere aufgrund der sich ändernden Streuung an der Oberfläche, und damit ein gleichmäßiger Koagulationsprozess erreicht.

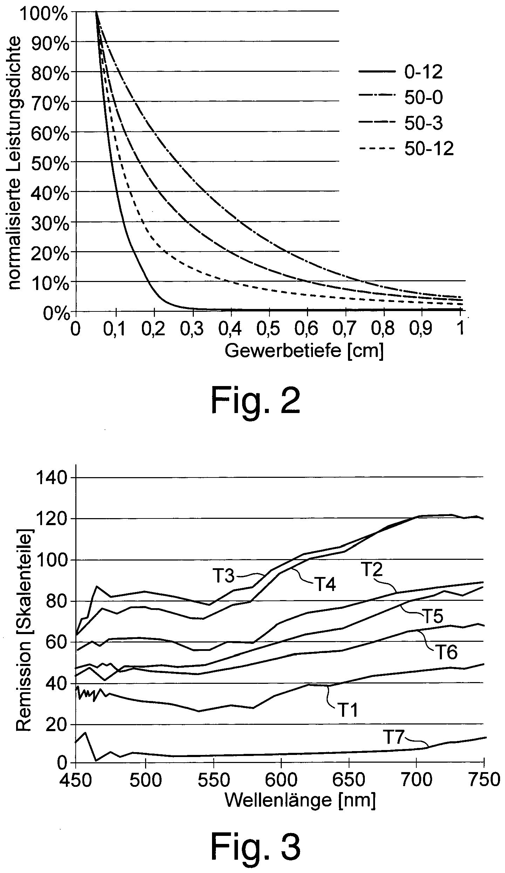

Dabei wird ausgenutzt, dass die Einstellung der effektiven optischen Eindringtiefe in einem weiten Bereich über die Änderung des Mischungsverhältnisses der einzelnen Anteile erfolgen kann, wobei die einzelnen spektralen Komponenten unterschiedliche Absorptions- und Streukoeffizienten in Gewebe und Kleber aufweisen. Figur 2 zeigt hierzu ein Beispiel für den Einfluss der Mischung zweier Wellenlängen am Beispiel der Eindringtiefe in Haut. Durch Änderung der Leistungsanteile zweier Laserwellenlängen von (0 W, 12 W) über (50 W, 12 W) und (50 W, 3 W) zu (50 W, 0 W) kann die effektive optische Eindringtiefe von etwa 0,12 mm auf 0,4 mm erhöht werden.

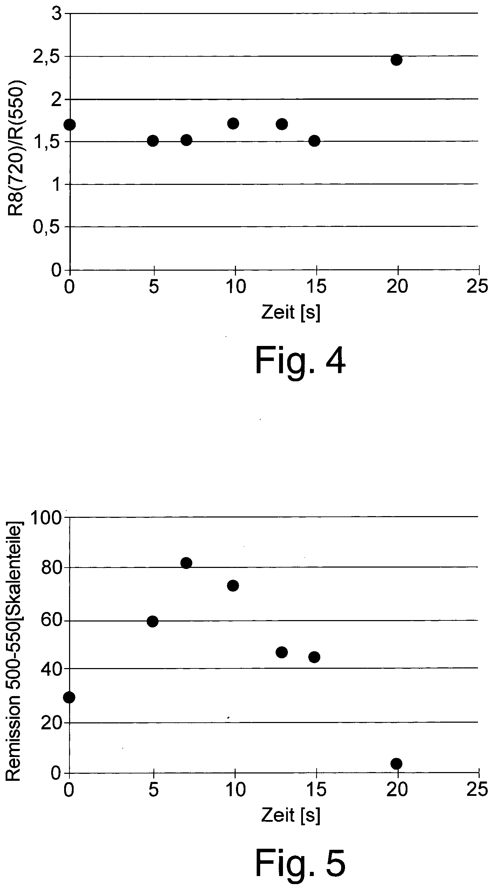

Der Detektor 5 für die Erfassung des Rückstreusignals kann als passiver Detektor ausgestaltet sein, der aus lichtempfindlichen Elementen, wie Photomultiplier, Photodioden oder CCD-Arrays besteht. Es kann jedoch auch ein aktiver Detektor eingesetzt werden, der darüber hinaus über eine oder mehrere Lichtquellen 13, bspw. Halogenlampen oder LEDs, zur Beleuchtung der Bearbeitungsoberfläche verfügt. Damit kann zusätzlich auch die spektrale Verteilung des rückgestreuten Lichtes über einen größeren Spektralbereich analysiert werden. So kann das Rückstreusignal spektral und zeitlich analysiert werden, um daraus ein Steuersignal abzuleiten, das die spektrale Leistungsverteilung der Laserstrahlquellen dynamisch an den Koagulationsprozess anpasst. In Figur 3 ist hierzu beispielhaft das Remissionsspektrum zu verschiedenen Zeitpunkten während der Koagulation von Muskelgewebe des Schweins dargestellt. Die Zeitpunkte T1 bis T7 entsprechen hierbei: T1 = 0s (nativ), T2 = 5s, T3 = 7s, T4 = 10s, T5 = 13s, T6 = 15s und T7 = 20s (karbonisiert). Das Rückstreusignal steigt dabei mit zunehmender Koagulationsdauer zunächst vorwiegend im roten Spektralbereich an (T2, T3) um dann mit zunehmender Gewebeänderung auf Werte unterhalb des nativen Anfangswertes abzusinken. Bei längerer Bestrahlung ist die Karbonisation zum Zeitpunkt T7 am Absinken des Remissionsgrads insgesamt sowie der relativen Erhöhung des infraroten Anteils im Vergleich zum grünen Anteil zu erkennen. Dies ist in der Figur 4 verdeutlicht, die den Quotienten der Remission im Spektralbereich von 720 nm zu 550 nm mit zunehmender Koagulationsdauer darstellt. Das Auftreten der Karbonisation bei t = 20s ist durch den Anstieg des Quotienten erkennbar. Figur 5 zeigt die zeitliche Änderung der Remission im Wellenlängenintervall von 500 bis 550 nm mit zunehmender Koagulationsdauer.

Mit der vorgeschlagenen Vorrichtung kann eine Anpassung der Absorptionslänge an spezifische Aufgabenstellungen bei geeigneter Wahl der beiden Wellenlängen in weiten Grenzen erfolgen, ohne dass ein Zusatz extrinsischer Absorber notwendig ist. Die effektive optische Eindringtiefe kann an unterschiedliche Dicken und optische Eigenschaften der Wundauflagen und vor allem optimal an den jeweiligen Koagulationszustand angepasst werden. Auch die Tiefe einer Koagulation ist durch die Wahl der Wellenlängen in weiten Grenzen wählbar.

Die vorliegende Erfindung beschreibt somit eine Vorrichtung, mit der die zeitgleiche Bereitstellung und örtliche Überlagerung mindestens zweier unterschiedlicher Wellenlängen zur Thermokoagulation ermöglicht wird. Die spektralen Leistungsanteile werden entsprechend einem vorgegebenen zeitlichen Verlauf gesteuert oder dynamisch entsprechend den Prozesssignalen angepasst. Damit kann die spektrale Leistung der Laserquelle dynamisch an veränderliche optische Eigenschaften des Gewebes angepasst werden, so dass die effektive optische Eindringtiefe in Körpergewebe und Wundauflagen kontrolliert werden kann. Die Vorrichtung ist besonders für die Koagulation von Proteinklebern zur Fixierung von Wundauflagen, den Verschluss von Hautwunden, die Gefäßanastomose, zur Blutstillung und zur Koagulation von Körpergewebe geeignet.

Bezugszeichenliste

| 1 | erste steuerbare Stromquelle |
|---|---|
| 2 | erster Laserbarren |
| 3 | zweite steuerbare Stromquelle |
| 4 | zweiter Laserbarren |
| 5 | Detektor |
| 6 | dichroitischer Spiegel |
| 7 | Einkoppeloptik |
| 8 | Lichtleitfaser |
| 9 | Kollimationsoptik |
| 10 | Bearbeitungslaserstrahl |
| 11 | Rückstreusignal |
| 12 | Steuereinrichtung |
| 13 | zusätzliche Lichtquelle |
| 14 | Wundauflage |
| 15 | Kleberschicht |
| 16 | Gewebe |

**Patentansprüche**

1. Vorrichtung zur Thermokoagulation von körpergewebe (16) mittels Laserstrahlung mit

   - mindestens zwei Laserstrahlquellen (2, 4), die Laserstrahlung mit unterschiedlicher Zentralwellenlänge auf eine Bearbeitungsoberfläche emittieren,
   - einer Einrichtung (6-8) zur Überlagerung der Laserstrahlung der Laserstrahlquellen (2,4), und
   - einer Steuereinrichtung (12) zur Steuerung der Laserleistung der jeweiligen Laserstrahlquellen (2,4), die die Laserstrahlquellen (2,4) in einem Bearbeitungsmodus der Vorrichtung zur gleichzeitigen Emission der Laserstrahlung für die Durchführung eines Thermokoagulationsprozesses ansteuert,
   **dadurch gekennzeichnet,**
   **dass** die Vorrichtung eine Einrichtung (5) zur Erfassung von Rückstreusignalen von der Bearbeitungsoberfläche als Prozesssignale aufweist, wobei die Einrichtung (5) zur Erfassung von Rückstreusignalen die Rückstreusignale (11) spektral aufgelöst oder die Rückstreusignale (11) in zwei unterschiedlichen Spektralbereichen getrennt voneinander erfasst, und
   **dass** die Steuereinrichtung (12) so ausgebildet ist, dass sie das Verhältnis der Laserleistungen der Laserstrahlquellen (2,4) im Bearbeitungsmodus in Abhängigkeit des aus dem Thermokoagulationsprozess erfassten Prozesssignals von den erfassten Rückstreusignalen auf Basis einer spektralen Analyse der Rückstreuung oder auf Basis der Stärke der in jedem Spektralbereich gemessenen Rückstreuung dynamisch ändert.

2. Vorrichtung nach Anspruch 1,
   **dadurch gekennzeichnet,**
   **dass** zur Thermokoagulation von biologischen Gewebe die mindestens zwei Laserstrahlquellen (2,4) so gewählt sind, dass die Zentralwellenlänge einer ersten (2) der Laserstrahlquellen (2,4) von dem biologischen Gewebe um mindestens einen Faktor 4 stärker absorbiert wird als die Zentralwellenlänge einer zweiten (4) der Laserstrahlquellen (2,4).

3. Vorrichtung nach Anspruch 1,
   **dadurch gekennzeichnet,**
   **dass** eine erste (2) der mindestens zwei Laserstrahlquellen (2,4) die Zentralwellenlänge bei > 1250 nm und eine zweite (4) der mindestens zwei Laserstrahlquellen (2,4) die Zentralwellenlänge bei < 1250 nm aufweist.

4. Vorrichtung nach einem der Ansprüche 1 bis 3,
**dadurch gekennzeichnet,**
**dass** die Laserstrahlquellen (2,4) Diodenlaser sind.

5. Vorrichtung nach einem der Ansprüche 1 bis 4,
**dadurch gekennzeichnet,**
**dass** die Einrichtung (6-8) zur Überlagerung der Laserstrahlung der Laserstrahlquellen (2,4) eine Lichtleitfaser (8) umfasst, in die die Laserstrahlung der Laserstrahlquellen (2,4) eingekoppelt wird.

6. Vorrichtung nach einem der Ansprüche 1 bis 5,
**dadurch gekennzeichnet,**
**dass** die Einrichtung (5) zur Erfassung von Rückstreusignalen eine oder mehrere zusätzliche Lichtquellen (13) zur Bestrahlung der Bearbeitungsoberfläche aufweist.

7. Vorrichtung nach einem der Ansprüche 1 bis 6,
**dadurch gekennzeichnet,**
**dass** die Steuereinrichtung (12) so ausgebildet ist, dass sie die Laserleistung der Laserstrahlquelle (4) mit der kürzeren Zentralwellenlänge bei Erhöhung der Rückstreuung erhöht und gleichzeitig die Laserleistung der Laserstrahlquelle (4) mit der längeren Zentralwellenlänge verringert.

8. Vorrichtung nach einem der Ansprüche 1 bis 7,
**dadurch gekennzeichnet,**
**dass** die Vorrichtung eine Einrichtung zur Erfassung einer Temperatur einer Bearbeitungsoberfläche aufweist und die Steuereinrichtung (12) eine Gesamtlaserleistung der Laserstrahlquellen (2,4) in Abhängigkeit von der erfassten Temperatur regelt.


**Claims**

1. Apparatus for thermocoagulation of body tissue (16) by means of laser radiation with

   - at least two laser beam sources (2, 4) which emit laser radiation having different central wavelengths towards a processing surface,
   - a device (6-8) for superimposing the laser radiation of the laser beam sources (2,4), and
   - a control device (12) for controlling the laser power of the respective laser beam sources (2, 4), which actuates the laser beam sources (2, 4) in a processing mode of the apparatus for the simultaneous emission of the laser radiation in order to carry out a thermocoagulation process,
   **characterized in that**
   the apparatus is equipped with a device (5) for detecting backscatter signals from the processing surface as process signals,
   wherein the device (5) for detecting backscatter signals detects the backscatter signals (11) in spectrally resolved manner or detects the backscatter signals (11) separately from each other in two different spectral ranges, and the control device (12) is designed such that it changes the ratio of the laser powers of the laser beam sources (2, 4) dynamically in the processing mode depending on the process signal of the detected backscatter signals detected from the thermocoagulation process based on a spectral analysis of the backscatter or based on the strength of the backscatter measured in each spectral range.

2. Apparatus according to Claim 1,
**characterized in that**
for the purpose of thermocoagulation of biological tissue the at least two laser beam sources (2, 4) are selected such that the central wavelength of a first (2) of the laser beam sources (2, 4) is absorbed by the biological tissue at least four times stronger than the central wavelength a second (4) of the laser beam sources (2, 4).

3. Apparatus according to Claim 1,
**characterized in that**
a first (2) of the at least two laser beam sources (2, 4) has the central wavelength at >1250 nm and a second (4) of the at least two laser beam sources (2, 4) has the central wavelength at <1250 nm.

4. Apparatus according to any one of Claims 1 to 3,
**characterized in that**
the laser beam sources (2, 4) are diode lasers.

5. Apparatus according to any one of Claims 1 to 4,
**characterized in that**
the device (6-8) for superimposing the laser radiation of the laser beam sources (2, 4) comprises an optical fibre (8) into which the laser radiation of the laser beam sources (2, 4) is coupled.

6. Apparatus according to any one of Claims 1 to 5,
**characterized in that**
the device (5) for detecting backscatter signals comprises one or more additional light sources (13) for irradiation of the processing surface.

7. Apparatus according to any one of Claims 1 to 6,
**characterized in that**
the control device (12) is designed such that it increases the laser power of the laser beam source (4) having the shorter central wavelength when the backscatter increases, and simultaneously reduces the laser power of the laser beam source (4) having the longer central wavelength.

8. Apparatus according to any one of Claims 1 to 7,
**characterized in that**
the apparatus comprises a device for detecting a temperature of a processing surface, and the control device (12) regulates a total laser power of the laser beam sources (2, 4) depending on the temperature detected.


**Revendications**

1. Dispositif de thermocoagulation de tissu corporel (16) au moyen d'un rayonnement laser comportant

   - au moins deux sources de faisceau laser (2, 4), qui émettent un rayonnement laser de différentes longueurs d'ondes centrales sur une surface de traitement,
   - un dispositif (6-8) pour superposer le rayonnement laser des sources de faisceau laser (2,4) et
   - un dispositif de commande (12) pour contrôler la puissance laser des sources de faisceau laser respectives (2,4), qui commande des sources de faisceaux laser (2,4) dans un mode de traitement du dispositif d'émission simultanée du rayonnement laser pour la mise en œuvre d'un procédé de thermocoagulation,
   **caractérisé en ce que**
   le dispositif comprend un dispositif (5) pour détecter les signaux de rétrodiffusion à partir de la surface de traitement en tant que signaux de processus,
   dans lequel le dispositif (5) pour détecter des signaux de rétrodiffusion résout spectralement les signaux de rétrodiffusion (11) ou détecte les signaux de rétrodiffusion (11) séparément les uns des autres dans deux gammes spectrales différentes, et
   **en ce que** le dispositif de commande (12) est conçu de telle sorte qu'il modifie dynamiquement le rapport des puissances laser des sources de faisceau laser (2, 4) en mode de traitement en fonction du signal de processus enregistré à partir du processus de thermocoagulation détecté par les signaux de rétrodiffusion sur la base d'une analyse spectrale de la rétrodiffusion ou sur la base de l'intensité de la rétrodiffusion mesurée dans chaque gamme spectrale.

2. Dispositif selon la revendication 1,
   **caractérisé en ce que**
   pour la thermocoagulation des tissus biologiques, les au moins deux sources de faisceau laser (2, 4) sont choisies de telle sorte que la longueur d'onde centrale d'une première (2) des sources de faisceau laser (2, 4) soit absorbée par le tissu biologique par au moins un facteur 4 de plus que la longueur d'onde centrale d'une deuxième (4) des sources de faisceau laser (2, 4).

3. Dispositif selon la revendication 1,
   **caractérisé en ce qu'**
   une première (2) des au moins deux sources de faisceau laser (2, 4) a la longueur d'onde centrale à >1250 nm et une seconde (4) des au moins deux sources de faisceau laser (2, 4) a la longueur d'onde centrale à <1250 nm.

4. Dispositif selon une des revendications 1 à 3,
   **caractérisé en ce que**
   les sources de faisceau laser (2, 4) sont des diodes laser.

5. Dispositif selon une des revendications 1 à 4,
   **caractérisé en ce que**
   le dispositif (6-8) de superposition du rayonnement laser des sources de faisceau laser (2, 4) comprend une fibre optique (8), dans laquelle le rayonnement laser des sources de faisceau laser (2, 4) est injecté.

6. Dispositif selon une des revendications 1 à 5,
   **caractérisé en ce que**
   le dispositif (5) de détection des signaux de rétrodiffusion présente une ou plusieurs sources de lumière supplémentaires (13) pour irradier la surface de traitement.

7. Dispositif selon une des revendications 1 à 6,
   **caractérisé en ce que**
   le dispositif de commande (12) est conçu pour qu'il augmente la puissance laser de la source de faisceau laser (4) avec la longueur d'onde centrale plus courte lorsque la rétrodiffusion augmente et diminue simultanément la puissance laser de la source de faisceau laser (4) avec la longueur d'onde centrale plus longue.

8. Dispositif selon une des revendications 1 à 7,
   **caractérisé en ce que**
   le dispositif comporte un dispositif de détection de la température d'une surface de traitement et le dispositif de commande (12) régule une puissance laser totale des sources de faisceau laser (2, 4) en fonction de la température enregistrée.

Fig. 1

## Fig. 2

## Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070179484 A1
- US 2012296323 A
- US 2003109860 A
- WO 2011143663 A2

**In der Beschreibung aufgeführte Nicht-Patentliteratur**

- **I. GABAY et al.** Temperature-Controlled Two-Wavelength Laser Soldering of Tissues. *Lasers in Surgery and Medicine,* 2011, vol. 43, 907-913